# EUROPÄISCHE PATENTSCHRIFT

(11) **EP 2 102 142 B1**
(45) Veröffentlichungstag und Bekanntmachung des Hinweises auf die Patenterteilung: **07.07.2010**
(21) Anmeldenummer: 07803144.0
(22) Anmeldetag: 03.09.2007
(51) Int. Cl.: C07C 51/56, C07C 51/573

(54) **VERBESSERTES VERFAHREN ZUR HERSTELLUNG VON UNGESÄTTIGTEN CARBONSÄUREANHYDRIDEN**
IMPROVED METHOD FOR THE PRODUCTION OF UNSATURATED CARBOXYLIC ACID ANHYDRIDES
PROCÉDÉ AMÉLIORÉ PERMETTANT DE PRODUIRE DES ANHYDRIDES D'ACIDES CARBOXYLIQUES INSATURÉS

(30) Priorität: 18.12.2006 DE 102006060162
(43) Veröffentlichungstag der Anmeldung: 23.09.2009
(73) Patentinhaber: Evonik Röhm GmbH, 64293 Darmstadt (DE)
(72) Erfinder: BRÖLL, Dirk, 63225 Langen (DE)
(86) Internationale Anmeldenummer: PCT/EP2007/059157
(87) Internationale Veröffentlichungsnummer: WO 2008/074523

(56) Entgegenhaltungen:
- EP-A- 0 196 520
- DE-A1- 3 644 222
- DE-B3-102006 029 320
- US-A- 4 857 239

## Beschreibung

Die Erfindung beschreibt ein verbessertes Verfahren zur Herstellung von ungesättigten Carbonsäureanhydriden, insbesondere die Reaktion einer ungesättigten Carbonsäure mit einem niedermolekularen aliphatischen Carbonsäureanhydrid.

In DE-A-3510035 wird ein Verfahren zur Herstellung von ungesättigten Carbonsäureanhydriden durch säurekatalysierte Umanhydridisierungsreaktion von Essigsäureanhydrid mit einer ungesättigten Carbonsäure im Mittelteil einer Destillationskolonne beschrieben. Als Katalysator werden homogene Katalysatoren angegeben, vorzugsweise Schwefelsäure, aliphatische bzw. aromatische Sulfonsäuren oder Phosphorsäure. Zur Erreichung eines vollständigen Umsatzes wird Essigsäureanhydrid in einem Überschuss von 0.1 bis 0.5 Mol pro Mol Carbonsäure eingesetzt, wobei dann am Kolonnenkopf eine Mischung aus Essigsäure und Essigsäureanhydrid anfällt, also keine reine Essigsäure gewonnen wird.

Darüber hinaus wird das Produkt verunreinigt durch den Katalysator gebildet, der erst in einem weiteren Verfahrensschritt entfernt werden muss.

In US-A- 4,857,239 wird ein Verfahren zur Herstellung von Methacrylsäureanhydrid beschrieben, wobei das Molverhältnis von Methacrylsäure zu Essigsäureanhydrid 2.1 bis 3 beträgt und ein Polymerisationsinhibitor in die Destillationskolonne zugegeben wird. Nachteilig ist, dass das im Überschuss eingesetzte Edukt ungenutzt anfällt. Außerdem wird kein die Reaktion beschleunigender Katalysator eingesetzt.

In US-A-2003/0018217 wird ein Verfahren zur Herstellung von Methacrylsäureanhydrid beschrieben, wobei das anfängliche Molverhältnis von Methacrylsäure zu Essigsäureanhydrid bevorzugt 9 bis 11 beträgt. Die entstehende Essigsäure wird sofort abgeführt und der freiwerdende Reaktorinhalt mit Essigsäureanhydrid aufgefüllt. Zur Vermeidung einer Polymerisation werden in den Reaktor und in die Kolonne Inhibitoren zugegeben. Es werden viele Nebenprodukte gebildet, die sich nicht vollständig entfernen lassen. Als Nebenprodukt tritt auch das Zwischenprodukt Acetyl(meth)acrylat (Mischanhydrid aus Essigsäureanhydrid und (Meth)acrylsäureanhydrid) auf. Auch hier wird kein Katalysator eingesetzt.

Aufgabe ist es nun ein verbessertes Verfahren zur Herstellung von ungesättigten Carbonsäureanhydriden bereitzustellen, bei dem ein stöchiometrischer Überschuss eines der Edukte vermieden wird, die Bildung von Nebenkomponenten möglichst unterdrückt wird und ein vollständiger Umsatz der Edukte erzielt wird. Darüber hinaus soll die Polymerisation in allen Bereichen weitgehend vermieden werden und die Raum-Zeit-Ausbeute der Reaktion erhöht werden.

Gegenstand der Erfindung ist ein verbessertes Verfahren zur Herstellung von ungesättigten Carbonsäureanhydriden der allgemeinen Formel I

R-C(O)-O-C(O)-R (I),

in der R einen ungesättigten organischen Rest mit 2 bis 12 C-Atomen darstellt,
durch Umanhydridisierung von einem aliphatischen Carbonsäureanhydrid mit einer Carbonsäure der allgemeinen Formel II

R-COOH (II),

in der R die oben angegebene Bedeutung hat,
in einer Apparatur, bestehend aus einem Reaktionsbereich und einer Rektifikationskolonne, dadurch gekennzeichnet, dass
a) die Edukte in stöchiometrischen Verhältnissen in einen Reaktionsbereich eingespeist werden,
b) die als Nebenprodukt entstehende Carbonsäure dem Reaktionsgemisch entzogen wird,
c) anschließend das gebildete Carbonsäureanhydrid der Formel 1 abgezogen wird,
d) die nicht umgesetzten Edukte in den Reaktionsbereich zurückgeführt werden,
e) der Reaktionsbereich einen heterogenen Katalysator enthält und
f) ein oder mehrere Polymerisationsinhibitoren zugegeben werden.

Durch diese technischen Merkmale wird erreicht, dass ein vollständiger Umsatz der Edukte erzielt wird, die Raum-Zeit-Ausbeute deutlich gesteigert wird und die Polymerisation in allen Bereichen weitgehend vermieden wird. Außerdem ist eine Katalysatorabtrennung in einem weiteren Trennapparat unnötig.

Für das erfindungsgemäße Verfahren geeignete Carbonsäuren weisen einen ungesättigten organischen Rest mit 2 bis 12, vorzugsweise 2 bis 6, besonders bevorzugt 2 bis 4 Kohlenstoffatomen auf. Geeignete Alkenylgruppen sind die Vinyl-, Allyl-, 2-Methyl-2-propen-, 2-Butenyl-, 2-Pentenyl-, 2-Decenyl, 1-Undecenyl und die 9,12-Octadecadienyl-Gruppe. Besonders bevorzugt sind die Vinyl- und die Allylgruppe.

Zu den besonders bevorzugten Carbonsäuren gehören u.a. (Meth)acrylsäuren. Der Begriff (Meth)acrylsäuren ist in der Fachwelt bekannt, wobei hierunter neben Acrylsäure und Methacrylsäure auch Derivate dieser Säuren zu verstehen sind. Zu diesen Derivaten gehören unter anderem β-Methylacrylsäure (Butensäure, Crotonsäure), α, β-Dimethylacrylsäure, β-Ethylacrylsäure, α-Chloracrylsäure, α-Cyanacrylsäure, 1-(Trifluormethyl)acrylsäure, sowie β,β-Dimethylacrylsäure. Bevorzugt sind Acrylsäure (Propensäure) und Methacrylsäure (2-Methylpropensäure).

Geeignete Carbonsäureanhydride für das erfindungsgemäße Verfahren sind der Fachwelt ebenfalls bekannt. Bevorzugte Verbindungen besitzen die allgemeine

Formel III R'-C(O)-O-C(O)-R' (III),

worin R' einen C₁ bis C₄-Alkylrest darstellt. Vorzugsweise wird Essigsäureanhydrid verwendet.

Erfindungsgemäß ist unter einem stöchiometrischen Verhältnis ein molares Verhältnis von 1,9 bis 2,1 zu 1 von Carbonsäure zu Carbonsäureanhydrid zu verstehen.

Für die Abtrennung der Produktströme gemäß der vorliegenden Erfindung kann jede Rektifikationskolonne verwendet werden, die zwischen 5 bis 50 Trennstufen besitzt. Bevorzugt beträgt die Zahl der Trennstufen 20 bis 30. Als Anzahl der Trennstufen wird in der vorliegenden Erfindung die Anzahl der Böden bei einer Bodenkolonne multipliziert mit dem Bodenwirkungsgrad oder die Anzahl der theoretischen Trennstufen im Fall einer Packungskolonne oder eine Kolonne mit Füllkörpern bezeichnet.

Beispiele für eine Rektifikationskolonne mit Böden beinhalten solche wie Glockenböden, Siebböden, Tunnelböden, Ventilböden, Schlitzböden, Sieb-Schlitzböden, Sieb-Glockenböden, Düsenböden, Zentrifugalböden, für eine Rektifikationskolonne mit Füllkörpern solche wie Raschig-Ringe, Lessing-Ringe, Pall-Ringe, Berl-Sättel, Intalox Sättel und für eine Rektifikationskolonne mit Packungen solche wie vom Typ Mellapak (Sulzer), Rombopak (Kühni), Montz-Pak (Montz) und Packungen mit Katalysatortaschen, beispielsweise Katapak (Sulzer).

Eine Rektifikationskolonne mit Kombinationen aus Bereichen von Böden, aus Bereichen von Füllkörpern und/oder aus Bereichen von Packungen kann ebenso verwendet werden.

Bevorzugt wird eine Rektifikationskolonne, mit Füllkörpern und/oder Packungen eingesetzt.

Die Rektifikationskolonne kann aus jedem hierfür geeigneten Material hergestellt werden. Hierzu gehören u.a. Edelstahl sowie inerte Materialien.

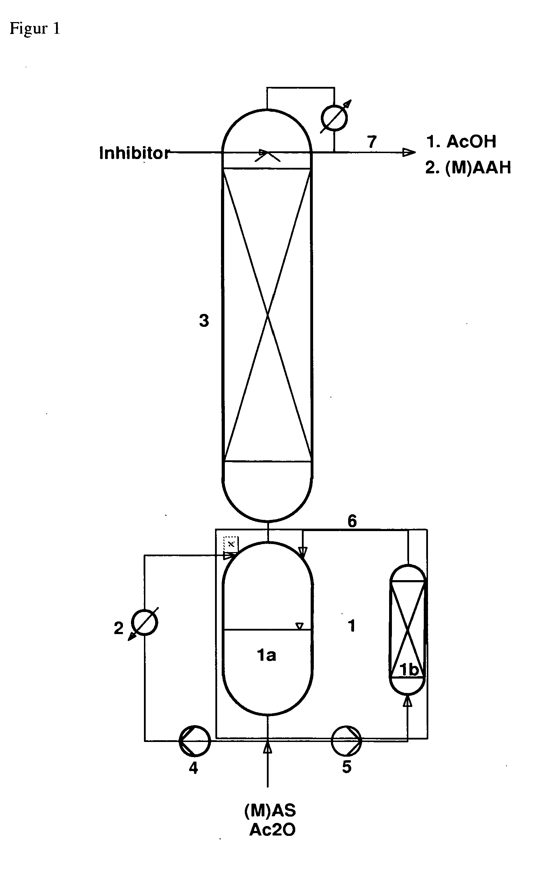

Die Apparatur weist mindestens einen Bereich, nachfolgend Reaktionsbereich genannt, auf, in dem vorzugsweise mindestens ein Katalysator vorgesehen ist. Dieser Reaktionsbereich kann innerhalb und/oder außerhalb der Rektifikationskolonne liegen. Vorzugsweise wird dieser Reaktionsbereich jedoch außerhalb der Rektifikationskolonne angeordnet, wobei eine dieser bevorzugten Ausführungsformen in Figur 1 näher erläutert wird.

Die Reaktion wird bevorzugt bei Temperaturen im Bereich von 30 bis 120 °C, besonders bevorzugt bei 40 bis 100 °C, insbesondere bei 50 bis 80 °C, durchgeführt. Dabei ist die Reaktionstemperatur abhängig vom eingestellten Systemdruck. Bei einer Anordnung des Reaktionsbereich innerhalb der Kolonne wird die Reaktion vorzugsweise im Druckbereich von 5 bis 100 mbar (absolut), insbesondere bei 10 bis 50 mbar (absolut) und besonders bevorzugt bei 20 bis 40 mbar (absolut) durchgeführt.

Falls sich der Reaktionsbereich außerhalb der Kolonne befindet, können dort andere Druck- und Temperaturverhältnisse gewählt werden als in der Kolonne. Das hat den Vorteil, dass die Reaktionsparameter des Reaktors unabhängig von den Betriebsbedingungen in der Kolonne eingestellt werden können.

Bei der Herstellung von (Meth)acrylsäureanhydrid aus Essigsäureanhydrid und (Meth)acrylsäure beträgt die Reaktionstemperatur vorzugsweise 40 bis 100 °C, besonders bevorzugt 50 bis 90 °C und ganz besonders bevorzugt 70 bis 85 °C.

Die Reaktionsmischung kann neben den Reaktanten weitere Bestandteile, wie beispielsweise Lösungsmittel, Katalysatoren und Polymerisationsinhibitoren umfassen.

Bevorzugt werden in dem Reaktionsbereich heterogene Katalysatoren verwendet. Als heterogene Katalysatoren besonders geeignet sind saure Festbettkatalysatoren, insbesondere saure Ionenaustauscher.

Überraschenderweise wurde mit diesen sauren Ionenaustauschern gefunden, dass die Bildung des Zwischenproduktes Acetyl(meth)acrylat (Mischanhydrid aus Essigsäureanhydrid und (Meth)acrylsäureanhydrid) minimiert wird und nur in Spuren im Carbonsäureanhydrid der Formel 1 vorliegt.

Zu den besonders geeigneten sauren Ionenaustauschern gehören insbesondere Kationenaustauscherharze wie sulfonsäuregruppenhaltige Styrol-Divinylbenzolpolymere. Geeignete Kationenaustauscherharze können kommerziell von Rohm&Haas unter der Handelsbezeichnung Amberlyst^{®}, von Dow unter der Handelsbezeichung Dowex^{®} und von Lanxess unter der Handelsbezeichnung Lewatit^{®} erhalten werden.

Die Katalysatormenge in L beträgt vorzugsweise 1/100 bis zu 1/1 des Reaktionsbereiches, besonders bevorzugt 1/30 bis 1/5.

Zu den bevorzugt einsetzbaren Polymerisationsinhibitoren gehören unter anderem Octadecyl-3-(3,5-di-tert-butyl-4-hydroxyphenyl)-propionat, Phenothiazin, Hydrochinon, Hydrochinonmonomethylether, 4-Hydroxy-2,2,6,6-tetramethylpiperidinooxyl (TEMPOL), 2,4-Dimethyl-6-tert-butylphenol, 2,6-Di-tert-butylphenol, 2,6-Di-tert-butyl-4-methylphenol, para-substituierte Phenylendiamine wie z. B. N,N'-Diphenyl-p-phenylendiamin, 1,4-Benzochinon, 2,6-Di-tert-butyl-alpha-(dimethylamino)-p-cresol, 2,5-Di-tert-butylhydrochinon oder Gemische aus zwei oder mehreren dieser Stabilisatoren. Ganz besonders bevorzugt ist Phenothiazin.

Die Zudosierung des Inhibitors kann in den Feed vor den Reaktionsbereich und/oder in die Rektifikationskolonne, vorzugsweise an deren Kopf, erfolgen.

Erfindungsgemäß erfolgt die Umanhydridisierung in einer Apparatur, in der die Edukte in den Reaktionsbereich eingespeist werden. Die neu gebildete Carbonsäure, als leichtest siedende Reaktionskomponente, wird am Kopf der Rektifikationskolonne bevorzugt bei vermindertem Druck abgezogen. Nicht umgesetzte Edukte und gebildete Zwischenprodukte werden in den Reaktionsbereich zurückführt, zum Beispiel mittels einer Pumpe.

Nachdem die neu gebildete Carbonsäure vollständig abgezogen wurde, wird anschließend das ungesättigte Carbonsäureanhydrid der Formel I am Kopf der Rektifikationskolonne bevorzugt bei vermindertem Druck abgezogen.

Der Katalysator wird in einem separaten Teil oder im ganzen Reaktionsbereich der Apparatur bereitgestellt. Eine separate Anordnung des Katalysators ist bevorzugt, wobei nicht umgesetzte Edukte und gebildete Zwischenprodukte in den mit Katalysator gefüllten Teil zurückführt werden. Hierdurch entsteht kontinuierlich das ungesättigte Carbonsäureanhydrid, beispielsweise (Meth)acrylsäureanhydrid, sowie die neu gebildete Carbonsäure, beispielsweise Essigsäure.

Eine bevorzugte Ausführungsform des erfindungsgemäßen Verfahrens ist in Figur 1 schematisch dargelegt. Der Reaktionsbereich (1) ist in Figur 1 grau hinterlegt und beinhaltet den Hauptteil des Reaktionsbereiches (1a), den separaten Teil des Reaktionsbereiches (1 b) sowie die zu- und abführenden Rohrleitungen.

(Meth)acrylsäure (=(M)AS)) und Essigsäureanhydrid (=Ac₂O) werden zunächst in den Hauptteil des Reaktionsbereiches (1 a) eingespeist.

Anschließend wird die gesamte Anlage unter verminderten Druck gesetzt und die Reaktionsmischung im Hauptteil des Reaktionsbereiches mit Hilfe eines Wärmeaustauschers (2) erwärmt, bis sich die über dem Hauptteil des Reaktionsbereiches befindliche Rektifikationskolonne (3) vollständig mit Dampf und Flüssigkeit gefüllt hat.

Der Wärmeaustauscher (2) ist vorzugsweise separat angebracht und mit dem Hauptteil des Reaktionsbereiches (1 a) über Rohrleitungen verbunden. Die Reaktionsmischung wird vorzugsweise mit einer Pumpe (4) über den Wärmaustauscher (2) kontinuierlich gepumpt.

Nun wird die Pumpe zum separaten Teil des Reaktionsbereiches (5) gestartet und die Reaktionsmischung auch kontinuierlich über den separaten Teil des Reaktionsbereiches (1b) geführt.

Der separate Teil ist vorzugsweise ein Strömungsrohrreaktor, der einen sauren Festbettkatalysator, besonders bevorzugt einen sauren Ionenaustauscher enthält.

Der Abstrom (6) wird in den Hauptteil des Reaktionsbereiches (1 a) wieder zurückgeführt.

In der Rektifikationskolonne (3) findet die Trennung der Komponenten statt. Zur Vermeidung von Polymerisation wird vorzugsweise am Kopf der Kolonne Inhibitor zudosiert.

Am Kopf der Rektifikationskolonne (7) wird zunächst die gebildete Essigsäure als leichtest siedende Komponente abgezogen. Anschließend erfolgt der Abzug des gebildeten (Meth)acrylsäureanhydrids.

Nachfolgend soll die vorliegende Erfindung anhand von Beispielen näher erläutert werden.

### Beispiel 1: Herstellung von Methacrylsäureanhydrid

Für die Herstellung von Methacrylsäureanhydrid durch Reaktion von Methacrylsäure und Essigsäureanhydrid wurde eine Versuchsanlage gemäß Figur 1 aufgebaut.

Die Rektifikationskolonne (3) hatte ca. 25 Trennstufen. Diese Kolonne war mit Kopfkondensator ca. 3,5 m hoch, hatte einen Innendurchmesser von 100 mm und war mit Packungen der Fa. Sulzer, Typ CY bestückt. Als Polymerisationsinhibitor wurde Phenothiazin eingesetzt. Die Austräge am Kolonnenkopf (7) sowie die Heizleistung des Wärmetauschers (2) wurden durch Einstellung geeigneter Temperaturen und internen Kreislaufströmen in der Versuchsanlage geregelt. Der Hauptteil des Reaktionsbereiches (1 a) war ein Glasbehälter mit einem Volumen von 10 L. Als Wärmetauscher (2) wurde ein Fallfilmverdampfer eingesetzt.

Der Versuch erfolgte sowohl ohne (Versuch 1.1) als auch mit heterogenem Festbettkatalysator (Versuch 1.2) im separaten Teil des Reaktionsbereiches (1 b). Als heterogener Festbettkatalysator wurden 500 mL des sauren Ionenaustauschers Lewatit K2431 der Fa. Lanxess verwendet. Der Ionenaustauscher wurde vor Versuchsbeginn mit Methacrylsäure gewaschen, um das im Ionenaustauscher befindliche Wasser herauszuspülen. Die Methacrylsäure wurde anschließend weitestgehend aus dem Ionenaustauscher durch einen Filter abgesaugt.

Methacrylsäure und Essigsäureanhydrid wurden zunächst in den Hauptteil des Reaktionsbereiches (1 a) eingespeist. Anschließend wurde der Druck in der Anlage auf 20 bar am Kolonenkopf verringert und die Reaktionsmischung mit Hilfe einer Pumpe (4) kontinuierlich über den Wärmetauscher (2) geführt und erwärmt. Nachdem sich die Rektifikationskolonne vollständig mit Dampf und Flüssigkeit gefüllt hatte, wurden die Pumpe (5), die zum separaten Teil des Reaktionsbereiches (1 b) führt und der Austrag am Kolonnenkopf (7) gestartet.

Die folgende Tabelle zeigt die erhaltenen Versuchsergebnisse im Vergleich:

| Versuch | | 1.1 | 1.2 |
|---|---|---|---|
| MAS-Einsatz | mol | 72,92 | 72,92 |
| Ac2O-Einsatz | mol | 36,46 | 36,46 |
| MAS-Einsatz | kg | 6,28 | 6,28 |
| Ac2O-Einsatz | kg | 3,72 | 3,72 |
| Menge Ionenaustauscher | mL | 0 | 500 |
| Zeit bis zum Starten der Pumpe (0) | h | 1,0 | 1,0 |
| Zeit AcOH-Abzug | h | 8,5 | 2,0 |
| Reinheit AcOH-Abzug | | | |
| Essigsäure | wt-% | 74,21 | 93,14 |
| Essigsäureanhydrid | wt-% | 6,04 | 1,32 |
| Methacrylsäure | wt-% | 13,44 | 3,15 |
| Acetylmethacrylat | wt-% | 4,86 | 1,39 |
| Methacrylsäureanhydrid | wt-% | 1,44 | 1,00 |
| Menge AcOH-Abzug | kg | 5,23 | 4,59 |
| Zeit MAAH-Abzug | h | 3,0 | 2,5 |
| Reinheit MAAH-Abzug | | | |
| Essigsäure | wt-% | 0,06 | 0,04 |
| Essigsäureanhydrid | wt-% | 0,22 | 0,18 |
| Methacrylsäure | wt-% | 0,45 | 0,39 |
| Acetylmethacrylat | wt-% | 0,80 | 0,23 |
| Methacrylsäureanhydrid | wt-% | 96,35 | 98,91 |
| Hochsieder | wt-% | 2,12 | 0,25 |
| Menge MAAH-Abzug | kg | 4,49 | 5,18 |
| Ausbeute MAAH-Abzug | % | 79,8 | 92,1 |
| Rückstand im Reaktionsbereich (1) nach Versuchsende | kg | 0,28 | 0,23 |

Bei Verwendung des Ionenaustauschers (Versuch 1.2)
a) entstanden deutlich weniger Nebenprodukte (Hochsieder und Acetylmethacrylat),
b) konnte die Versuchszeit von insgesamt 12,5 h auf 5,5 h verringert werden,
c) waren weniger nichtumgesetzte Edukte (Ac2O und MAS) im AcOH-Abzug und MAAH-Abzug,
d) war die Ausbeute wesentlich höher.

### Beispiel 2: Herstellung von Acrylsäureanhydrid

Für die Herstellung von Acrylsäureanhydrid durch Reaktion von Acrylsäure und Essigsäureanhydrid wurde dieselbe Versuchsanlage wie in Beispiel 1 erläutert verwendet.

Die Versuchsbedingungen und die Vorgehensweise waren identisch mit den Angaben, die in Beispiel 1 angegeben sind.

Der Versuch erfolgte sowohl ohne (Versuch 2.1) als auch mit heterogenem Festbettkatalysator (Versuch 2.2) im separaten Teil des Reaktionsbereiches (1 b). Als heterogener Festbettkatalysator wurden 500 mL des sauren Ionenaustauschers Lewatit K2431 der Fa. Lanxess verwendet. Der Ionenaustauscher wurde vor Versuchsbeginn mit Acrylsäure gewaschen, um das im Ionenaustauscher befindliche Wasser herauszuspülen. Die Acrylsäure wurde anschließend weitestgehend aus dem Ionenaustauscher durch einen Filter abgesaugt.

Die folgende Tabelle zeigt die erhaltenen Versuchsergebnisse im Vergleich.

| Versuch | | 2.1 | 2.2 |
|---|---|---|---|
| AS-Einsatz | mol | 81,23 | 81,23 |
| Ac2O-Einsatz | mol | 40,62 | 40,62 |
| AS-Einsatz | kg | 5,85 | 5,85 |
| Ac2O-Einsatz | kg | 4,15 | 4,15 |
| Menge Ionenaustauscher | mL | 0 | 500 |
| Zeit bis zum Starten der Pumpe (0) | h | 1 | 1 |
| Zeit AcOH-Abzug | h | 10 | 2,25 |
| Reinheit AcOH-Abzug | | | |
| Essigsäure | wt-% | 72,15 | 90,03 |
| Essigsäureanhydrid | wt-% | 7,71 | 2,81 |
| Acrylsäure | wt-% | 13,94 | 4,79 |
| Acetylacrylat | wt-% | 4,86 | 1,32 |
| Acrylsäureanhydrid | wt-% | 1,35 | 1,06 |
| Menge AcOH-Abzug | kg | 5,82 | 5,19 |
| Zeit AAH-Abzug | h | 2,35 | 2,0 |
| Reinheit AAH-Abzug | | | |
| Essigsäure | wt-% | 0,07 | 0,03 |
| Essigsäureanhydrid | wt-% | 0,25 | 0,23 |
| Acrylsäure | wt-% | 0,51 | 0,50 |
| Acetylhacrylat | wt-% | 0,97 | 0,37 |
| Acrylsäureanhydrid | wt-% | 95,88 | 98,55 |
| Hochsieder | wt-% | 2,32 | 0,32 |
| Menge AAH-Abzug | kg | 3,92 | 4,58 |
| Ausbeute AAH-Abzug | % | 76,6 | 89,5 |
| Rückstand im Reaktionsbereich (1) nach Versuchsende | kg | 0,26 | 0,23 |

Bei Verwendung des Ionenaustauschers (Versuch 2.2)
e) entstanden deutlich weniger Nebenprodukte (Hochsieder und Acetylacrylat),
f) konnte die Versuchszeit von insgesamt 13,35 h auf 5,25 h verringert werden,
g) waren weniger nichtumgesetzte Edukte (Ac2O und AS) im AcOH-Abzug und AAH-Abzug,
h) war die Ausbeute wesentlich höher.

## Patentansprüche

1. Verbessertes Verfahren zur Herstellung von ungesättigten Carbonsäureanhydriden der allgemeinen Formel I
R-C(O)-O-C(O)-R (I),
in der R einen ungesättigten organischen Rest mit 2 bis 12 C-Atomen darstellt,
durch Umanhydridisierung von einem aliphatischen Carbonsäureanhydrid mit einer Carbonsäure der allgemeinen Formel II
R-COOH (II),
in der R die oben angegebene Bedeutung hat,
in einer Apparatur, bestehend aus einem Reaktionsbereich und einer Rektifikationskolonne, **dadurch gekennzeichnet, dass**
a) die Edukte in stöchiometrischen Verhältnissen in einen Reaktionsbereich eingespeist werden,
b) die als Nebenprodukt entstehende Carbonsäure dem Reaktionsgemisch entzogen wird,
c) anschließend das gebildete Carbonsäureanhydrid der Formel 1 abgezogen wird,
d) die nicht umgesetzten Edukte in den Reaktionsbereich zurückgeführt werden,
e) der Reaktionsbereich einen heterogenen Katalysator enthält und
f) ein oder mehrere Polymerisationsinhibitoren zugegeben werden.

2. Verfahren gemäß Anspruch 1, **dadurch gekennzeichnet, dass** im gesamten Reaktionsbereich oder in einem Teil des Reaktionsbereichs ein heterogener Katalysator verwendet wird.

3. Verfahren gemäß Anspruch 2, **dadurch gekennzeichnet, dass** ein saurer Festbettkatalysator verwendet wird.

4. Verfahren gemäß Anspruch 2 oder 3, **dadurch gekennzeichnet, dass** ein Kationenaustauscher als Katalysator verwendet wird.

5. Verfahren gemäß einem der Ansprüche 1 bis 4, **dadurch gekennzeichnet, dass** sich der Katalysator in einem separaten Bereich des Reaktionsbereiches befindet.

6. Verfahren gemäß einem der Ansprüche 1 bis 5, **dadurch gekennzeichnet, dass** das ungesättigte Carbonsäureanhydrid der Formel I (Meth)acrylsäureanhydrid ist, hergestellt durch Umanhydridisierung von Essigsäureanhydrid und (Meth)acrylsäure.

## Claims

1. Improved process for preparing unsaturated carboxylic anhydrides of the general formula I
R-C(O)-O-C(O)-R (I)
in which R is an unsaturated organic radical having 2 to 12 carbon atoms
by transanhydridization of an aliphatic carboxylic anhydride with a carboxylic acid of the general formula II
R-COOH (II)
in which R is as defined above
in an apparatus consisting of a reaction region and a rectification column, **characterized in that**
a) the reactants are fed into a reaction region in stoichiometric ratios,
b) the carboxylic acid formed as a by-product is removed from the reaction mixture,
c) then the carboxylic anhydride of the formula 1 formed is drawn off,
d) the unconverted reactants are recycled into the reaction region,
e) the reaction region contains a heterogeneous catalyst and
f) one or more polymerization inhibitors are added.

2. Process according to Claim 1, **characterized in that** a heterogeneous catalyst is used in the entire reaction region or in part of the reaction region.

3. Process according to Claim 2, **characterized in that** an acidic fixed bed catalyst is used.

4. Process according to Claim 2 or 3, **characterized in that** a cationic exchanger is used as the catalyst.

5. Process according to one of Claims 1 to 4, **characterized in that** the catalyst is in a separate region of the reaction region.

6. Process according to one of Claims 1 to 5, **characterized in that** the unsaturated carboxylic anhydride of the formula I is (meth)acrylic anhydride, prepared by transanhydridization of acetic anhydride and (meth)acrylic acid.

## Revendications

1. Procédé amélioré pour la production d'anhydrides d'acides carboxyliques insaturés de formule générale I
R-C(O)-O-C(O)-R (I),
dans laquelle R représente un radical organique insaturé ayant de 2 à 12 atomes de carbone,
par trans-anhydrisation d'un anhydride d'acide carboxylique aliphatique avec un acide carboxylique de formule générale II
R-COOH (II)
dans laquelle R a la signification donnée ci-dessus,
dans un appareil qui est constitué d'une zone de réaction et d'une colonne de rectification, **caractérisé en ce que**
a) les produits de départ sont introduits en rapports stoechiométriques dans une zone de réaction,
b) l'acide carboxylique formé en tant que sous-produit est prélevé du mélange de réaction,
c) ensuite l'anhydride d'acide carboxylique formé de formule I est retiré,
d) les produits de départ n'ayant pas réagi sont renvoyés dans la zone de réaction,
e) la zone de réaction contient un catalyseur hétérogène et
f) un ou plusieurs inhibiteurs de polymérisation sont ajoutés.

2. Procédé selon la revendication 1, **caractérisé en ce qu'**on utilise un catalyseur hétérogène dans toute la zone de réaction ou dans une partie de la zone de réaction.

3. Procédé selon la revendication 2, **caractérisé en ce qu'**on utilise un catalyseur acide en lit fixe.

4. Procédé selon la revendication 2 ou 3, **caractérisé en ce qu'**on utilise comme catalyseur un échangeur de cations.

5. Procédé selon l'une quelconque des revendications 1 à 4, **caractérisé en ce que** le catalyseur se trouve dans une zone séparée de la zone de réaction.

6. Procédé selon l'une quelconque des revendications 1 à 5, **caractérisé en ce que** l'anhydride d'acide carboxylique insaturé de formule I est l'anhydride (méth)acrylique, produit par trans-anhydrisation d'anhydride acétique et d'acide (méth)acrylique.
